# EUROPÄISCHE PATENTSCHRIFT

(11) **EP 4 489 676 B1**
(45) Veröffentlichungstag und Bekanntmachung des Hinweises auf die Patenterteilung: **09.07.2025**
(21) Anmeldenummer: 24725405.5
(22) Anmeldetag: 25.04.2024
(51) Int. Cl.: A61B 18/14, A61B 18/00

(54) **ELEKTROCHIRURGISCHES INSTRUMENT SOWIE ELEKTRODENANORDNUNG UND BASISGERÄT DAFÜR**
ELECTROSURGICAL INSTRUMENT, AND ALSO ELECTRODE ARRANGEMENT AND BASE DEVICE FOR IT
INSTRUMENT ÉLECTROCHIRURGICAL, ET AGENCEMENT D'ÉLECTRODE ET DISPOSITIF DE BASE POUR CELUI-CI

(30) Priorität: 24.05.2023 DE 102023113613
(43) Veröffentlichungstag der Anmeldung: 15.01.2025
(73) Patentinhaber: BOWA-electronic GmbH & Co. KG, 72810 Gomaringen (DE)
(72) Erfinder: KIESSLING, Ingo, 72131 Ofterdingen (DE); PRANTNER, Michael, 72116 Mössingen (DE)
(74) Vertreter: Schneider, Peter Christian
(86) Internationale Anmeldenummer: PCT/EP2024/061455
(87) Internationale Veröffentlichungsnummer: WO 2024/240443

(56) Entgegenhaltungen:
- WO-A1-2015/032496
- DE-A1- 102012 009 058
- US-A1- 2005 010 080
- US-A1- 2014 364 847

## Beschreibung

### Gebiet der Erfindung

Die Erfindung betrifft ein elektrochirurgisches Instrument, umfassend
- ein Basisgerät mit einer die Form eines gerade erstreckten Kanals aufweisenden Aufnahme für einen Steckerabschnitt einer Elektrodenanordnung und
- eine Elektrodenanordnung mit einem sich zwischen einem distalen und einem proximalen Ende erstreckenden Gestänge, dessen distales Ende einen elektrochirurgischen Effektor trägt oder mit einem solchen koppelbar ist und dessen proximales Ende als ein Steckerabschnitt ausgebildet und dichtend in die Aufnahme des Basisgerätes eingesteckt ist,

wobei die Aufnahme des Basisgerätes und der Steckerabschnitt der Elektrodenanordnung jeweils eine Mehrzahl voneinander beabstandeter, gegeneinander elektrisch isolierter Kontaktelemente tragen, die eine entsprechende Mehrzahl von Kontaktpaaren bilden, welche jeweils ein steckerseitiges und ein aufnahmeseitiges Kontaktelement, die einander elektrisch kontaktieren, umfassen,
und wobei der Steckerabschnitt eine Manschette aus einem elastischen Dichtungsmaterial aufweist, deren Außenwandung dichtend an der Innenwandung eines Dichtabschnitts der Aufnahme anliegt.

Die Erfindung betrifft weiter eine Elektrodenanordnung für ein elektrochirurgisches Instrument, umfassend ein sich zwischen einem distalen und einem proximalen Ende erstreckendes Gestänge, dessen distales Ende einen elektrochirurgischen Effektor trägt oder mit einem solchen koppelbar ist und dessen proximales Ende als ein Steckerabschnitt ausgebildet ist, der beabstandet voneinander einer Mehrzahl gegeneinander elektrisch isolierter, steckerseitiger Kontaktelemente trägt, die über gegeneinander elektrisch isolierte Elektrodenzuleitungen mit Elektroden des Effektors verbunden oder verbindbar sind.

Die Erfindung betrifft schließlich ein Basisgerät für ein elektrochirurgisches Instrument, umfassend eine die Form eines gerade erstreckten Kanals aufweisende Aufnahme für einen Steckerabschnitt einer Elektrodenanordnung,
wobei die Aufnahme beabstandet voneinander eine Mehrzahl gegeneinander elektrisch isolierter, aufnahmeseitiger Kontaktelemente trägt, die über gegeneinander elektrisch isolierte Kontaktzuleitungen mit vom Äußeren des Basisgerätes her zugänglichen Versorgungsanschlüssen verbunden oder verbindbar sind.

### Stand der Technik

Derartige elektrochirurgische Instrumente und Elektrodenanordnungen bzw. Basisgeräte dafür sind bekannt aus der DE 103 17 038 A1. Ferner sind ähnliche Vorrichtungen aus US 2014/364847 A1 bekannt.

Elektrochirurgieinstrumente, wie beispielsweise zum elektrochirurgischen Resezieren, finden in der modernen Medizin vielfach Einsatz. Bekannte Einsatzgebiete sind die endoskopische Resektion, Verdampfung oder elektrische Koagulation von Körpergewebe. Hierzu wird ein hohler Schaft des Instrumentes durch eine operativ erzeugte oder natürlich vorhandene Körperöffnung in das Innere des Patientenkörpers eingeführt. Durch den Schaft hindurch wird eine Elektrodeneinheit in das Körperinnere eingeführt, wobei die Elektrodeneinheit und der Schaft über ein oft als ein Griffstück ausgebildetes Basisgerät funktional miteinander verbunden sind. Eine im Basisgerät angeordnete, vom Operateur bedarfsweise betätigbare, meist im Basisgerät integrierte mechanische Anordnung ist eingerichtet, die Elektrodeneinheit gezielt relativ zum Schaft axial zu verschieben. Hierdurch ist eine präzise Positionierung der Spitze, d. h. des distalen, mit einem speziell für den geplanten Eingriff gestalteten Effektor versehenen Endes der Elektrodeneinheit auf der zu behandelnden Gewebestelle möglich. Eine ebenfalls vom Operateur bedarfsweise betätigbare und ebenfalls meist im Basisgerät integrierte elektrische Anordnung dient der elektrischen Verbindung der Elektrodeneinheit mit einem an das Instrument angeschlossenen Hochspannungsgenerator und der gezielten Beaufschlagung der Elektrodeneinheit mit einer vom Generator gelieferten Hochspannung.

Aus der eingangs genannten, gattungsbildenden DE 103 17 038 A1 ist ein elektrochirurgisches Instrument bekannt, dessen überwiegend aus elektrisch nicht-leitendem Kunststoff aufgebautes Basisgerät zur mechanischen Halterung und elektrischen Kontaktierung einer Elektrodenanordnung eine Aufnahme aufweist, die als gerade erstreckter Kanal mit über seine gesamte Länge im Wesentlichen konstantem Querschnitt ausgebildete. An zwei axial voneinander beabstandeten Positionen ist die Innenwandung des Kanals mit Kontaktblechen versehen. An diese aufnahmeseitigen Kontaktelemente sind außerhalb des Kanals verlaufende Leiter angeschlossen, die ihrerseits zu einem elektrischen Versorgungskabel führen, mittels dessen das Basisgerät über eine Steuereinheit an den Generator angeschlossen werden kann. Das proximale Ende der Elektrodenanordnung ist als ein zu besagter Aufnahme korrespondierender Stecker ausgebildet. Dieser führt in seinem Inneren zwei mit unterschiedlichen Elektroden des Effektors am distalen Ende der Elektrodenanordnung verbundene Leiter, die an axial voneinander beabstandeten Positionen die Oberfläche des Steckers durchbrechen bzw. diese dort lokal bilden. Zwischen diesen steckerseitigen Kontaktelementen ist die Außenseite des Steckers aus elektrisch nicht-leitendem Material aufgebaut. Dieser Aufbau ist auch von sog. Klinkensteckern grundsätzlich bekannt. Die Positionen der aufnahme- und der steckerseitigen Kontaktelemente sind so aufeinander abgestimmt, dass sie im eingesteckten Zustand der Elektrodeneinheit einander paarweise kontaktieren, sodass eine Versorgung der Elektroden des Effektors mit der elektrischen Leistung des Generators ermöglicht wird. Um ein Eindringen von Flüssigkeit in den Spalt zwischen Aufnahme und Stecker zu vermeiden, sind in die Wandung der Aufnahme zwei Ringnuten mit gerundetem Nutprofil eingearbeitet, in die raumfüllend je ein Dichtring eingespritzt ist. Jeder Dichtring liegt an der Eingangsöffnung seiner Ringnut dichtend an der Außenfläche des Steckers an. Eine dieser Dichtungen ist distal des vorderen Kontaktpaares und die andere zwischen den beiden Kontaktpaaren angeordnet. Auf diese Weise soll ein Eindringen von Flüssigkeit, jedenfalls aber ein Kurzschluss zwischen den beiden Kontaktpaaren vermieden werden.

Dieses Konzept hat mehrere Nachteile. So ist es primär für nur einmal verwendbare Basisgeräte (sog. Disposables) geeignet. Die in die Ringnuten eingespritzten Dichtringe sind nicht austauschbar. Ihr Verschleiß bei mehrfachem Gebrauch würde mit der Zeit zu Undichtigkeiten und der Gefahr von Kurzschlüssen führen. Beim Einmalgebrauch besteht diese Gefahr hingegen nicht. Auch ist es keine Option, sie austauschbar, z.B. in Form klassischer O-Ringe, zu gestalten. Zum einen sind sie im Inneren der Aufnahme nahezu unzugänglich. Zum anderen beruht das Funktionsprinzip der O-Ring-Dichtung wesentlich darauf, dass Flüssigkeit zwischen den O-Ring und die Nutwand seiner (geradwandigen) Ringnut dringt; dies macht jedoch eine zuverlässige Reinigung nach Gebrauch schwierig.

Ein weiterer Nachteil des bekannten Konzeptes besteht darin, dass, um eine zuverlässige Dichtung zu erzielen, die Anlagekraft der Dichtringe am Steckerabschnitt der Elektrodenanordnung recht hoch sein muss. Dies bedeutet aber eine hohe Reibung, die bei jeder Axialbewegung des Steckerabschnitts in die Aufnahme, mithin beim Wechsel der Elektrodenanordnung überwunden werden muss. Das ist für den Benutzer lästig und kann sogar dazu führen, dass insbesondere der Einsteckvorgang vorzeitig, d. h. vor dem Erreichen der maximalen Einschubposition, das ggf. mit einer Verriegelung des Steckers in der Aufnahme korreliert sein kann, abgebrochen wird. Für einen sicheren Wechsel der Elektrodenanordnung wäre es daher hilfreich, ein System zu haben, bei dem eine nicht korrekt montierte Elektrodenanordnung ohne Weiteres sofort erkennbar ist, z. B. indem sie in auffallender Weise aus dem Basisgerät herausfällt oder sogar aktiv ausgestoßen wird.

### Aufgabenstellung

Es ist die Aufgabe der vorliegenden Erfindung, gattungsgemäße elektrochirurgische Instrumente sowie Elektrodenanordnungen und Basisgeräte dafür derart weiterzubilden, dass ein Wechsel der Elektrodenanordnung leichter und zuverlässiger vonstattengehen kann.

### Darlegung der Erfindung

Diese Aufgabe wird in Verbindung mit den Merkmalen des Oberbegriffs von Anspruch 1 dadurch gelöst, dass die Manschette axial zwischen einer steckerseitigen Fixierung und einer aufnahmeseitigen, quer zur Axialrichtung der Aufnahme erstreckten und nach distal weisenden Anlagefläche geklemmt ist.

Die Aufgabe wird weiter in Verbindung mit den Merkmalen des Oberbegriffs von Anspruch 11 dadurch gelöst, dass der Steckerabschnitt eine distal an einer steckerseitigen Anlage anliegende Manschette aus einem elastischen Dichtungsmaterial trägt,
- deren Innenwandung an der Außenwandung eines tragenden Bereichs des Steckerabschnitts anliegt und
- deren proximale Stirnwand eine senkrecht zu ihrer Axialrichtung stehende Dichtfläche bildet.

Die Aufgabe wird zudem in Verbindung mit den Merkmalen des Oberbegriffs von Anspruch 13 dadurch gelöst, dass der Steckerabschnitt als einen selbsttragenden Bereich seiner selbst eine Manschette aus einem elastischen Dichtungsmaterial aufweist, deren proximale Stirnwand eine senkrecht zur Axialrichtung der Manschette stehende Dichtfläche bildet.

Die Aufgabe wird schließlich in Verbindung mit den Merkmalen des Oberbegriffs von Anspruch 18 dadurch gelöst, dass die Aufnahme als proximale Begrenzung eines Dichtabschnitts, der zur dichtenden, radialen Anlage einer einen Bestandteil der Elektrodenanordnung bildenden Manschette aus einem elastischen Dichtungsmaterial geeignet ist, eine quer zu ihrer Axialrichtung erstreckte und nach distal weisende Anlagefläche zur dichtenden, axialen Anlage der proximalen Stirnwand besagter Manschette aufweist.

Die Begriffe "distal" und "proximal" dienen hier im Wesentlichen der Unterscheidung der beiden (einzigen) axialen Richtungen, sind aber angelehnt an die fachsprachlichen Bezeichnungen für "im Gebrauchsfall patientenseitig / vom Operateur entfernt" (distal) und "im Gebrauchsfall operateursseitig / näher zum Operateur liegend" (proximal).

Bevorzugte Ausführungsformen sind Gegenstand der abhängigen Ansprüche.

Der Kerngedanke der Erfindung besteht darin, die funktionale Dichtung zwischen Steckerabschnitt und Aufnahme erst im unmittelbaren zeitlichen Umfeld des Erreichens der Montageendstellung, d. h. bei etwa maximalem Einschub der Elektrodenanordnung in das Basisgerät, wirksam werden zu lassen. Die Manschette kann hinsichtlich ihrer radialen Ausdehnung relativ zur Aufnahme so dimensioniert werden, dass sie deren Innenfläche beim Einschieben nicht oder nur in leichtem Gleitkontakt berührt. Entsprechend niedrig sind die beim Einschieben zu überwindenden Reibungskräfte. Kurz vor Erreichen der maximalen Einschubtiefe jedoch kontaktiert die proximale Stirnwand der Manschette die korrespondierende Anlagefläche, welche den Dichtabschnitt der Aufnahme nach proximal begrenzt. Von da an führt ein tieferes Einschieben der distal steckerseitig fixierten Manschette zu deren axialer Kompression. Die dadurch in der Manschette entstehenden Kräfte führen zu einem radialen Ausweichen des elastischen Dichtungsmaterials, d. h. zu einer elastischen Verdickung der Manschette. Hierdurch legt sie sich im Bereich ihrer gesamten Mantelwand dichtend an die Innenwandung der Aufnahme an. Die entsprechende Anlagekraft führt jedoch nicht zu einer störenden Reibung, da in diesem Zustand quasi keine axiale Bewegung der Manschette relativ zur Aufnahme mehr erforderlich ist. In diesem Zustand kann die Elektrodenanordnung im Basisgerät fixiert werden. Die Kompression der Manschette und die resultierende Dichtwirkung bleiben erhalten. Zusätzlich zu der erläuterten Dichtung aufgrund der Wechselwirkung zwischen der Mantelwand der Manschette und der Innenwandung der Aufnahme führt auch die Wechselwirkung zwischen der proximalen Stirnwand der Manschette und der korrespondierenden Anlagefläche der Aufnahme zu einer Dichtwirkung. Aufgrund der großen dichtungswirksamen Fläche ist die resultierende Dichtwirkung in einem erfindungsgemäßen elektrochirurgischen Instrument deutlich verbessert gegenüber dem nur lokale Dichtringe verwendenden Stand der Technik. Trotzdem kann das Einschieben bzw. Herausziehen der (entriegelten) Elektrodenanordnung aus dem Basisgerät nahezu reibungslos erfolgen, was den Wechsel einer Elektrodenanordnung deutlich vereinfacht. Zudem lässt sich beim Einstecken einer Elektrodenanordnung das Erreichen der maximalen Einschubposition durch einen ausgeprägten Kraftsprung deutlich fühlen, was die korrekte Bedienung erleichtert.

Aufgrund der Quasi-Reibungsfreiheit beim Wechsel der Elektrodenanordnungen ist auch der Verschleiß insbesondere des elastischen Dichtungsmaterials deutlich verringert. Dies bedeutet, dass sowohl Elektrodenanordnung wie auch Basisgerät, sofern gewünscht, als wiederverwendbare Bauteile ausgelegt sein können. In jedem Fall aber weist das Basisgerät keinerlei verschleißanfällige, elastische Dichtungsmaterialien auf und kann wiederverwendbar gestaltet sein. Der Vorteil der Wiederverwendbarkeit ist bei einem Basisgerät noch wertvoller als bei einer Elektordenanordnung. Das Basisgerät ist nämlich in der Regel deutlich komplexer und daher teurer aufgebaut als Elektrodenanordnungen; diese werden häufig ohnehin als Einmal-Geräte ausgelegt, da insbesondere ihr Effektor aufgrund der bei Betrieb von ihm erzeugten Plasmabögen starker Belastung und starkem Verschleiß ausgesetzt ist.

Zur speziellen Gestaltung der Manschette haben sich zwei alternative Varianten als besonders vorteilhaft erwiesen. Bei einer ersten Alternative ist vorgesehen, dass die Innenwandung der Manschette an der Außenwandung eines tragenden Bereichs des Steckerabschnitts anliegt, d. h. insbesondere als ein zum übrigen Gestänge der Elektrodenanordnung separates Bauteil ausgebildet ist. Eine solche separate Manschette kann axial verschieblich auf dem Steckerabschnitt angeordnet sein. In jedem Fall aber hat sie distal an einer steckerseitigen Anlage anzuliegen. Gegen diese stützt sie sich distal ab, sobald ihre proximale Stirnwand an der korrespondierenden, aufnahmeseitigen Anlagefläche anschlägt.

Bei einer zweiten Alternative ist vorgesehen, dass der Steckerabschnitt die Manschette als einen selbsttragenden Bereich seiner selbst aufweist. Mit anderen Worten ist also wenigstens ein Abschnitt des Gestänges der Elektrodenanordnung aus elastischem Dichtungsmaterial gefertigt und bildet besagte Manschette, wobei die Materialwahl und Dimensionierung so zu erfolgen hat, dass das Gestänge unter den bei bestimmungsgemäßer Handhabung auftretenden Kräften keine übermäßige Verbiegung zeigt. Die distale Fixierung der Manschette kann beispielsweise über eine stoffschlüssige oder auch einteilig materialeinheitliche Verbindung mit dem übrigen Steckerabschnitt bzw. Gestänge der Elektrodenanordnung realisiert sein.

Im Kontext beider vorgenannter Alternativen kann vorgesehen sein, dass die Manschette die Form eines dickwandigen Hohlzylinders aufweist. Als dickwandig sind im vorliegenden Kontext Hohlzylinder zu verstehen, deren Wanddicke mehr als 10% ihres Durchmessers ausmacht. Bei einer solchen Gestaltung wird der zur Manschette korrespondierende Dichtabschnitt einen runden Querschnitt axial konstanten Durchmessers aufweisen, wobei die Durchmesser von Manschette und Dichtabschnitt im Sinne der oben erläuterten Dichtwirkung aufeinander abgestimmt zu dimensionieren sind. Denkbar sind auch nicht-runde Querschnittsformen, die eine zusätzliche Verdrehsicherung der Manschette (und ggf. - bei entsprechend drehgesicherter Kopplung mit dem Gestänge - auch der Elektrodenanordnung) im Basisgerät bieten. Einsetzbare Beispiele für nicht-runde Querschnittsformen sind etwa quadratisch, rechteckig, elliptisch oder blütenförmig mit zwei oder mehr Blütenblättern

Bezüglich der Anordnung der Kontaktpaare, d. h. der steckerseitigen Kontaktelemente einerseits und der aufnahmeseitigen Kontaktelemente andererseits, sind unterschiedliche Gestaltungsvarianten denkbar. Bei einer ersten bevorzugten Variante ist vorgesehen, dass zwei der steckerseitigen Kontaktelemente axial voneinander beabstandet sind und die Manschette axial zwischen ihnen angeordnet ist. Für das entsprechende Basisgerät bedeutet dies, dass die entsprechenden aufnahmeseitigen Kontaktelemente ebenfalls axial voneinander beabstandet sind und der Dichtabschnitt der Aufnahme axial zwischen ihnen liegt. Bei dem elektrochirurgischen Instrument im Montageendzustand führt dies dann dazu, dass die entsprechenden Kontaktpaare axial voneinander beabstandet sind und die Manschette bzw. der Dichtabschnitt axial zwischen ihnen angeordnet sind. Bei dieser Gestaltung wird also wesentlicher Wert darauf gelegt, dass durch Eindringen von Flüssigkeit kein Kurzschluss zwischen den beiden Kontaktpaaren entstehen kann. Das distale Kontaktpaar allerdings ist bei dieser Ausführungsform, sofern keine zusätzlichen Dichtungsmaßnahmen getroffen werden, durchaus von außen eindringender Flüssigkeit ausgesetzt. Eine solche Gestaltung empfiehlt sich daher insbesondere dann, wenn das distale Kontaktpaar elektrisch auf auf Neutralelektroden-Potential liegt. Das entsprechende steckerseitige Kontaktelement ist dann mit der Neutralelektrode des Effektors verbunden, welche eine große Kontaktfläche zu der den Effektor im Betriebsfall umspülenden Flüssigkeit aufweist. Die Aktivelektrode des Effektors ist mit dem steckerseitigen Kontaktelement des proximal der Manschette bzw. des Dichtabschnitts angeordneten Kontaktpaares verbunden.

Alternativ oder zusätzlich kann vorgesehen sein, dass zwei der Kontaktpaare azimutal, d. h. in Umfangsrichtung, voneinander beabstandet und proximal der Manschette angeordnet sind. Günstigerweise ist dabei zusätzlich vorgesehen, dass materialeinheitlich an die Manschette zwei Stege angeformt sind, die sich azimutal beidseitig zwischen besagten steckerseitigen Kontaktelementen axial bis zum proximalen Ende des Steckerabschnitts erstrecken und dieses ineinander übergehend umlaufen. Die Stege sind dabei vorzugsweise so dimensioniert, dass ihre äußeren Grate im Montageendzustand an der Innenwandung der Aufnahme anliegen. Bei dieser Ausführungsform befinden sich also zwei Kontaktpaare im durch die Manschette abgedichteten Bereich des Steckerabschnitts. Zugleich sind sie mittels der Stege auch noch gegeneinander in Umfangsrichtung abgedichtet, um selbst im Fall, dass Flüssigkeit die erfindungsgemäße Dichtung überwindet oder sich aufgrund unsachgemäßer Handhabung Flüssigkeit im proximalen Endbereich des Steckerabschnitts befindet, ein Kurzschluss zwischen den beiden Kontaktpaaren zuverlässig verhindert wird.

Bezüglich der speziellen Ausgestaltung der einzelnen Kontaktelemente ist eine Vielzahl von Varianten denkbar, die grundsätzlich auch von handelsüblichen Klinkensteckern bekannt sind. Als besonders vorteilhaft hat sich jedoch eine Variante erwiesen, bei der die Steckerelemente eines Kontaktpaares eine Doppelfunktion erfüllen. Primär dienen sie der elektrischen Kontaktierung. Sekundär können sie jedoch auch als mechanische Verriegelung dienen, mittels welcher die Elektrodenanordnung in Montageendstellung im Basisgerät fixiert wird. Hierzu weist der Steckerabschnitt eine Hinterschneidung mit einem nach distal weisenden Anschlag auf. An diesem kann im Montageendzustand ein bewegbares, in den Innenraum der Aufnahme ragendes Riegelelement des Basisgerätes anliegen. Das Rigelelement kann dabei radial und/oder tangential verschieblich und/oder verdrehbar ausgebildet ist. Auch ist die Ausgestaltung der Verriegelung nach dem Vorbild eines BNC-Steckers denkbar. Die Hinterschneidung des Steckerabschnittes kann beispielsweise als Ringnut oder lokale Ausnehmung, beispielsweise als Sackloch, ausgebildet sein. Bei dem Riegelelement, welches bevorzugt nach radial innen federvorgespannt ist, kann es sich um einen (in Bezug auf die Aufnahme bzw. den Steckerabschnitt) radial verschieblichen Stift handeln. Die Ausgestaltung der Hinterschneidung als Ringnut liefert einen rotativen Freiheitsgrad der Elektrodenanordnung relativ zum Basisgerät. Ist ein solcher Freiheitsgrad nicht gewünscht, kann er durch Ausgestaltung der Hinterschneidung als lokale Ausnehmung, in welche das Riegelelement hineinragt, unterbunden werden.

In beiden Fällen hat es sich als günstig erwiesen, die Hinterschneidung nach distal in Form einer Anlaufschräge auslaufen zu lassen. Wie der Fachmann verstehen wird, liegt das Riegelelement im Montageendzustand mit einer axial wirkenden Kraft am Anschlag der Hinterschneidung an, wobei diese Kraft durch die Rückstellkräfte des komprimierten elastischen Dichtungsmaterials der Manschette erzeugt werden. Unter solcher Krafteinwirkung kann es schwierig sein, zum Zwecke der Entriegelung des Riegelelements aus der Hinterschneidung nach radial außen zu verschieben. Eine Entlastung kann durch geringfügig tieferes Einschieben der Elektrodenanordnung in das Basisgerät entgegen den dabei weiter ansteigenden elastischen Kräften der Manschette erfolgen. Dieses weitere Einschieben würde durch eine senkrechte distale Begrenzung der Hinterschneidung unterbunden. Eine Anlaufschräge jedoch ermöglicht nicht nur diese Entlastungsbewegung, sondern überträgt zugleich eine nach radial außen wirkende Kraft auf das Riegelelement, welche dessen Rückzug aus der Aufnahme und daher die Entriegelung insgesamt unterstützt.

Weitere Einzelheiten und Vorteile der Erfindung ergeben sich aus der nachfolgenden speziellen Beschreibung und den Zeichnungen.

### Kurzbeschreibung der Zeichnungen

Es zeigen:
- Figur 1:: eine schematisierte, stark vereinfachte und ausschnittsweise Darstellung einer ersten Ausführungsform eines erfindungsgemäßen elektrochirurgischen Instrumentes (Teilfigur 1a), des proximalen Endes von dessen Elektrodenanordnung (Teilfigur 1b) sowie seines Basisgerätes (Teilfigur 1c),
- Figur 2:: eine schematisierte, ausschnittsweise Darstellung des proximalen Endes einer zweiten Ausführungsform einer erfindungsgemäßen Elektrodenanordnung,
- Figur 3:: eine schematisierte, ausschnittsweise Darstellung des proximalen Endes einer dritten Ausführungsform einer erfindungsgemäßen Elektrodenanordnung sowie
- Figur 4:: eine schematisierte, ausschnittsweise Darstellung des proximalen Endes einer vierten Ausführungsform einer erfindungsgemäßen Elektrodenanordnung.

### Beschreibung bevorzugter Ausführungsformen

Gleiche Bezugszeichen in den Figuren deuten auf gleiche oder analoge Elemente hin.

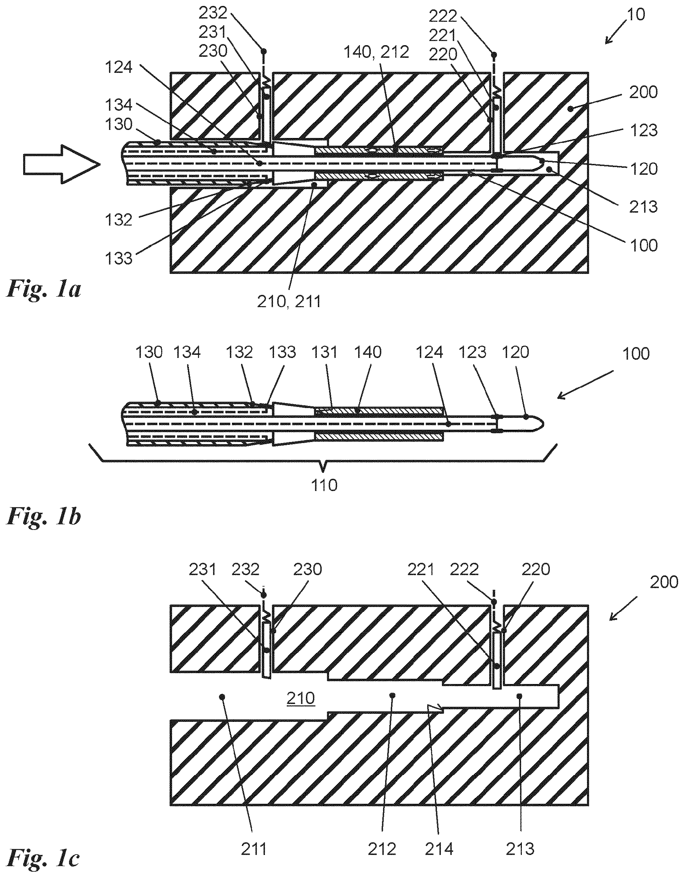

Figur 1a zeigt in stark vereinfachter, schematisierter und lediglich ausschnittsweiser Darstellung eine bevorzugte Ausführungsform eines erfindungsgemäßen elektrochirurgischen Instrumentes 10. Dieses umfasst im Wesentlichen zwei Hauptkomponenten, nämlich eine Elektrodenanordnung 100, deren distales Ende in Figur 1b separat gezeigt ist, und ein Basisgerät 200, welches - in extremer Vereinfachung im Wesentlichen kastenförmig dargestellt - in Figur 1c separat gezeigt ist.

Das Basisgerät 200 weist als hier relevanten Bestandteil eine Aufnahme 210 auf, die bei der gezeigten Ausführungsform in drei Abschnitte unterteilt ist. Die Aufnahme 210 kann bei der dargestellten Ausführungsform als ein im Wesentlichen rotationssymmetrisches Sackloch mit drei Abschnitten unterschiedlichen Durchmessers beschrieben werden. Der in Figur 1c links dargestellte, eingangsseitige bzw. distale Abschnitt mit größtem Durchmesser soll als Distalabschnitt 211 bezeichnet werden. Der in Figur 1c mittig dargestellte Abschnitt mittleren Durchmessers soll als Dichtungsabschnitt 212 bezeichnet werden. Der in Figur 1c rechts dargestellte Abschnitt kleinsten Durchmessers soll hier als Proximalabschnitt 213 bezeichnet werden. Die Abfolge unterschiedlicher Durchmesser hat insbesondere zur Konsequenz, dass an der Grenze zwischen Dichtungsabschnitt 212 und Proximalabschnitt 213 eine nach distal weisende Anlagefläche 214 entsteht.

Bei der dargestellten Ausführungsform ist das Basisgerät 200 mit zwei senkrecht zur Längserstreckung der Aufnahme 210 ausgerichteten Kontaktkanälen 220, 230 versehen, in welchen stiftartige Kontaktelemente 221, 231 axialbeweglich und federvorgespannt gelagert sind. Die Kontaktelemente 221, 231, die hier auch als Kontaktstifte 221, 231 bezeichnet werden sollen, sind jeweils über eine Kontaktzuleitung 222, 232 mit einem in den Figuren nicht dargestellten Versorgungsanschluss verbunden.

Figur 1b zeigt den als Steckerabschnitt 110 wirksamen, proximalen Endabschnitt der Elektrodenanordnung 100. Diese ist bei der dargestellten Ausführungsform koaxial aufgebaut. Sie weist insbesondere eine Innenstange 120 auf, die sich über ihre gesamte Länge erstreckt. Diese durchsetzt koaxial eine Außenstange 130, die kürzer gestaltet ist und insbesondere deutlich vor dem proximalen Ende der Innenstange 120 endet. Insbesondere wird das proximale Ende der Außenstange 130 durch eine senkrecht zur Axialrichtung ausgerichtete, steckerseitige Anlage 131 gebildet. Leicht distal von besagter steckerseitiger Anlage 131 beabstandet weist die Außenstange 130 eine Ringnut 132 auf. Die Innenstange 120 und die Außenstange 130 können aus elektrisch isolierendem oder elektrisch isoliertem Material ausgebildet sein und sind jedenfalls elektrisch gegeneinander isoliert. Jede der Stangen 120, 130 weist jedoch ein von außen zugängliches, steckerseitiges Kontaktelement 123 (proximal) bzw. 133 (distal) auf, welches über eine zugeordnete Elektrodenzuleitung 124 bzw. 134 mit einer in den Figuren nicht dargestellten, korrespondierenden Elektrode am distalen Ende der Elektrodenanordnung 100 verbunden ist. Bei der dargestellten Ausführungsform liegt das proximale steckerseitige Kontaktelement 123 der Innenstange 120 in deren Spitzenbereich; das distale steckerseitige Kontaktelement 133 der Außenstange 130 liegt im Bereich der Ringnut 132.

Bei der dargestellten Ausführungsform ist eine Manschette 140 aus einem elastischen, ggf. elektrisch isolierenden Dichtungsmaterial auf die Innenstange 120 aufgeschoben. Sie liegt mit ihrem distalen Ende an der steckerseitigen Anlage 131 an, die das proximale Ende der Außenstange 130 bildet. Die Manschette 140 hat beim gezeigten Ausführungsbeispiel die Form eines dickwandigen Hohlzylinders, dessen Innenfläche in leichtem Gleitkontakt auf der Außenfläche der Innenstange 120 axial verschieblich gelagert ist.

Zur Montage wird der Steckerabschnitt 110 der Elektrodenanordnung 100 (Figur 1b) in die Aufnahme 210 des Basisgerätes 200 (Fig. 1c) eingeschoben. Der eingeschobene Zustand ist in Figur 1a illustriert. Die Dimensionen sind so gewählt, dass insbesondere die Manschette 140 mit ihrer Außenfläche in leichtem Gleitkontakt zur Innenfläche des Dichtungsabschnitts 212 der Aufnahme 210 steht. Die Außendurchmesser der Innenstange 120 und der Außenstange 130 sind vorzugsweise kleiner als die Durchmesser des Proximalabschnitts 213 bzw. des Distalabschnitts 211 der Aufnahme 210 gestaltet, sodass die zum Einschieben nötige Kraft im Wesentlichen allein von dem (geringen) Reibkontakt zwischen der Manschette 140 und der Wandung des Dichtungsabschnitts 212 vorgegeben wird.

Nach einem nahezu vollständigen Einschieben der Elektrodenanordnung 100, insbesondere deren Steckerabschnitts 110 in die Aufnahme 210 schlägt das proximale Ende der Manschette 140 an der Anlagefläche 214 an, welche den Dichtungsabschnitt 212 der Aufnahme 210 gegen deren Proximalabschnitt 213 begrenzt. Ein weiteres Einschieben führt zu einer axialen Kompression der Manschette 140, was wiederum zu einer Dickenzunahme führt. Hierdurch legt sich die Außenfläche der Manschette 140 dichtend an die Innenfläche des Dichtungsabschnitts 212 der Aufnahme 210 an. Hierdurch wird eine zuverlässige Abdichtung insbesondere des Proximalbereichs 213 der Aufnahme bzw. des proximalen steckerseitigen Kontaktelementes 123 auf der Innenstange 120 der Elektrodenanordnung 100 gewährleistet.

Die axialen Positionen der steckerseitigen Kontaktelemente 123, 133 sind relativ zu den Kontaktkanälen 220 der Aufnahme 210 so gewählt, dass in dieser beschriebenen, vollständigen Einschubposition des Steckerabschnitts 110 die aufnahmeseitigen Kontaktelemente 221, 231 an den steckerseitigen Kontaktelementen 123, 133 anliegen. Wie oben bereits erläutert, befindet sich das distale steckerseitige Kontaktelement 133 im Bereich der Ringnut 132. Daher ragt auch der korrespondierende, distale Kontaktstift 231 in besagte Ringnut 132 hinein. Aufgrund der axialen Federkraft, die von der axial komprimierten Manschette 140 entwickelt wird, liegt der distale Kontaktstift 231 dabei insbesondere kraftbeaufschlagt an der proximalen Nutwand der Ringnut 132 an und verriegelt auf diese Weise den Steckerabschnitt 110 in der Aufnahme 210. Der distale Kontaktstift 231 erfüllt damit eine Doppelfunktion als Kontaktelement sowie als Riegelelement.

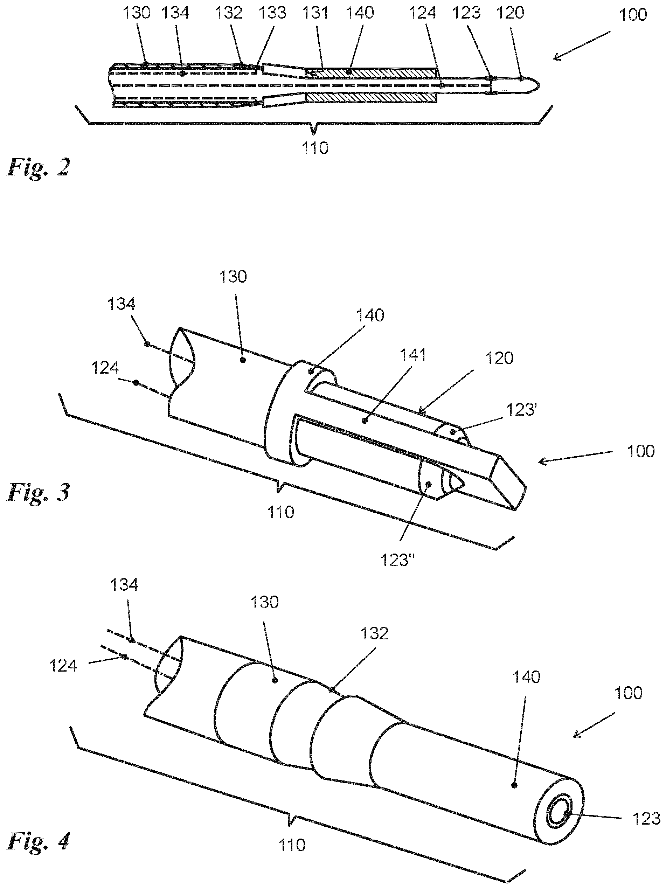

Figur 2 zeigt eine alternative Ausführungsform des Steckerabschnitts 110. Hier ist die Manschette 140 nicht als separates, auf die Innenstange 120 aufgeschobenes Bauteil ausgebildet, sondern stellt einen selbsttragenden Gestängeabschnitt dar, an den sich proximal die lediglich als Spitze ausgebildete Innenstange 120 und an den sich distal die Außenstange 130 anschließt. Im Übrigen kann auf die obige Erläuterung verwiesen werden.

Figur 3 zeigt eine weitere Ausführungsform des Steckerabschnitts 110 einer erfindungsgemäßen Elektrodenanordnung 100. Bei dieser ist die axiale Länge der eigentlichen Manschette 140 gegenüber den zuvor erläuterten Ausführungsformen stark reduziert. Allerdings sind proximal zwei Stege 141 (lediglich einer davon in Figur 3 erkennbar) an die Manschette 140 angeformt. Sie umlaufen insbesondere das proximale Ende der Innenstange 120 und unterteilen dieses somit in zwei azimutale Bereiche, die als elektrisch voneinander isolierte steckerseitige Kontaktelemente 123', 123" ausgebildet sein können. Dier Manschette 140 stellt deren Abdichtung gegenüber eindringender Flüssigkeit sicher; die Stege 141 stellen die Abdichtung der beiden steckerseitigen Kontaktelemente 123', 123" gegeneinander sicher. Die spezielle Formgebung der steckerseitigen Kontaktelemente 123', 123" kann weitgehend frei gewählt werden. Insbesondere können abgeflachte Formen, die eine bessere Anlage von aufnahmeseitigen Kontaktelementen ermöglichen, gewählt werden. Im Übrigen kann auf die vorangehende Erläuterung verwiesen werden.

Figur 4 schließlich zeigt eine weitere Ausführungsform des Steckerabschnitts 110 einer erfindungsgemäßen Elektrodenanordnung 100. Dieser zeichnet sich dadurch aus, dass die Innenstange 120 nicht über das proximale Ende der Manschette 140 hinausragt, sondern vielmehr selbst als Hohlstange ausgebildet ist, deren proximales Ende eine Buchse für ein korrespondierendes, aufnahmeseitiges Kontaktelement in Form eines Kontaktstiftes gestaltet ist. Im Übrigen kann auf die vorangehenden Erläuterungen verwiesen werden.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. So ist etwa eine Vervielfachung der erfindungsgemäßen Manschette denkbar, indem mehrere elastische und erfindungsgemäß komprimierte Manschetten in axialer Richtung hintereinander angeordnet sind. Zwischen je zwei solchen Manschetten kann ein steckerseitiges Kontaktelement angeordnet sein Die spezielle Gestaltung des distalen Endes der Elektrodenanordnung ist für die vorliegende Erfindung irrelevant. Auch die spezielle Ausgestaltung des Basisgerätes 200 ist, abgesehen von ihren erfindungsrelevanten Merkmalen im Bereich der Aufnahme 210, vom Fachmann weitgehend beliebig gestaltbar.

### Bezugszeichenliste

- 10: elektrochirurgisches Instrument
- 100: Elektrodenanordnung
- 110: Steckerabschnitt
- 120: Innenstange
- 123: proximales steckerseitiges Kontaktelement
- 123', 123": steckerseitiges Kontaktelemente
- 124: Elektrodenzuleitung
- 130: Außenstange
- 131: steckerseitige Anlage
- 132: Ringnut
- 133: distales steckerseitiges Kontaktelement
- 134: Elektrodenzuleitung
- 140: Manschette
- 141: Steg
- 200: Basisgerät
- 210: Aufnahme
- 211: Distalabschnitt von 210
- 212: Dichtungsabschnitt von 210
- 213: Proximalabschnitt von 210
- 214: aufnahmeseitige Anlagefläche
- 220: proximaler Kontaktkanal
- 221: proximales aufnahmeseitiges Kontaktelement / Kontaktstift
- 222: Kontaktzuleitung
- 230: distaler Kontaktkanal
- 231: distales aufnahmeseitiges Kontaktelement / Kontaktstift
- 232: Kontaktzuleitung

## Patentansprüche

1. Elektrochirurgisches Instrument (10), umfassend
- ein Basisgerät (200) mit einer die Form eines gerade erstreckten Kanals aufweisenden Aufnahme (210) für einen Steckerabschnitt (110) einer Elektrodenanordnung (100) und
- eine Elektrodenanordnung (100) mit einem sich zwischen einem distalen und einem proximalen Ende erstreckenden Gestänge, dessen distales Ende einen elektrochirurgischen Effektor trägt oder mit einem solchen koppelbar ist und dessen proximales Ende als ein Steckerabschnitt (110) ausgebildet und dichtend in die Aufnahme (210) des Basisgerätes (200) eingesteckt ist,
wobei die Aufnahme (210) des Basisgerätes (200) und der Steckerabschnitt (110) der Elektrodenanordnung (100) jeweils eine Mehrzahl voneinander beabstandeter, gegeneinander elektrisch isolierter Kontaktelemente (123, 123', 123", 133; 221, 231) tragen, die eine entsprechende Mehrzahl von Kontaktpaaren bilden, welche jeweils ein steckerseitiges und ein aufnahmeseitiges Kontaktelement (123, 123', 123", 133; 221, 231), die einander elektrisch kontaktieren, umfassen,
und wobei der Steckerabschnitt (110) eine Manschette (140) aus einem elastischen Dichtungsmaterial aufweist, deren Außenwandung dichtend an der Innenwandung eines Dichtabschnitts (212) der Aufnahme (210) anliegt,
**dadurch gekennzeichnet,**
**dass** die Manschette (140) axial zwischen einer steckerseitigen Fixierung und einer aufnahmeseitigen, quer zur Axialrichtung der Aufnahme (210) erstreckten und nach distal weisenden Anlagefläche (214) geklemmt ist.

2. Elektrochirurgisches Instrument (10) nach Anspruch 1,
**dadurch gekennzeichnet,**
**dass** die Innenwandung der Manschette (140) dichtend an der Außenwandung eines tragenden Bereichs des Steckerabschnitts (110) anliegt.

3. Elektrochirurgisches Instrument (10) nach Anspruch 1,
**dadurch gekennzeichnet,**
**dass** die Manschette (140) als ein selbsttragender Bereich des Steckerabschnitts (110) ausgebildet ist.

4. Elektrochirurgisches Instrument (10) nach einem der vorangehenden Ansprüche,
**dadurch gekennzeichnet,**
**dass** die Manschette (140) die Form eines dickwandigen Hohlzylinders aufweist und die Aufnahme (210) wenigstens im Bereich des Dichtabschnitts (212) einen runden Querschnitt axial konstanten Durchmessers aufweist.

5. Elektrochirurgisches Instrument (10) nach einem der vorangehenden Ansprüche,
**dadurch gekennzeichnet,**
**dass** der Steckerabschnitt (110) eine Hinterschneidung mit einem nach distal weisenden Anschlag aufweist, an dem ein bewegbares, in den Innenraum der Aufnahme ragendes Riegelelement anliegt.

6. Elektrochirurgisches Instrument (10) nach Anspruch 5,
**dadurch gekennzeichnet,**
**dass** die Hinterschneidung nach distal in Form einer Anlaufschräge ausläuft.

7. Elektrochirurgisches Instrument (10) nach einem der Ansprüche 5 bis 6,
**dadurch gekennzeichnet,**
**dass** die Hinterschneidung und das Riegelelement als die Kontaktelemente (231, 133) eines der Kontaktpaare ausgebildet sind.

8. Elektrochirurgisches Instrument (10) nach einem der vorangehenden Ansprüche,
**dadurch gekennzeichnet,**
**dass** zwei der Kontaktpaare axial voneinander beabstandet sind und die Manschette (140) axial zwischen ihnen angeordnet ist.

9. Elektrochirurgisches Instrument (10) nach einem der vorangehenden Ansprüche,
**dadurch gekennzeichnet,**
**dass** zwei der Kontaktpaare azimutal voneinander beabstandet und proximal der Manschette (140) angeordnet sind,
wobei materialeinheitlich an die Manschette (140) zwei Stege (141) angeformt sind, deren äußere Grate an der Innenwandung der Aufnahme (210) anliegen und sich azimutal beidseitig zwischen den Kontaktpaaren axial bis zum proximalen Ende des Steckerabschnitts (110) erstrecken und dieses ineinander übergehend umlaufen.

10. Elektrochirurgisches Instrument (10) nach einem der Ansprüche 1 bis 8,
**dadurch gekennzeichnet,**
**dass** eines der Kontaktpaare aus einem koaxial von proximal her in den Innenraum der Aufnahme (210), insbesondere deren Dichtungsabschnitt (212), ragenden Stift als aufnahmeseitigem Kontaktelement und einer korrespondierenden, koaxialen und in einer Öffnung am proximalen Ende der Elektrodenanordnung mündenden Buchse als steckerseitigem Kontaktelement (123) besteht.

11. Elektrodenanordnung (100) für ein elektrochirurgisches Instrument (10), umfassend ein sich zwischen einem distalen und einem proximalen Ende erstreckendes Gestänge, dessen distales Ende einen elektrochirurgischen Effektor trägt oder mit einem solchen koppelbar ist und dessen proximales Ende als ein Steckerabschnitt (110) ausgebildet ist, der beabstandet voneinander einer Mehrzahl gegeneinander elektrisch isolierter, steckerseitiger Kontaktelemente (123, 123', 123", 133) trägt, die über gegeneinander elektrisch isolierte Elektrodenzuleitungen (124, 134) mit Elektroden des Effektors verbunden oder verbindbar sind,
**dadurch gekennzeichnet,**
**dass** der Steckerabschnitt (110) eine distal an einer steckerseitigen Anlage (131) anliegende Manschette (140) aus einem elastischen Dichtungsmaterial trägt,
- deren Innenwandung an der Außenwandung eines tragenden Bereichs des Steckerabschnitts (110) anliegt und
- deren proximale Stirnwand eine senkrecht zu ihrer Axialrichtung stehende Dichtfläche bildet.

12. Elektrodenanordnung (100) nach Anspruch 11,
**dadurch gekennzeichnet,**
**dass** die Manschette (140) axial verschieblich auf dem Steckerabschnitt (110) angeordnet ist.

13. Elektrodenanordnung (100) für ein elektrochirurgisches Instrument (10), umfassend ein sich zwischen einem distalen und einem proximalen Ende erstreckendes Gestänge, dessen distales Ende einen elektrochirurgischen Effektor trägt oder mit einem solchen koppelbar ist und dessen proximales Ende als ein Steckerabschnitt (110) ausgebildet ist, der beabstandet voneinander einer Mehrzahl gegeneinander elektrisch isolierter, steckerseitiger Kontaktelemente (123, 123', 123", 133) trägt, die über gegeneinander elektrisch isolierte Elektrodenzuleitungen (124, 134) mit Elektroden des Effektors verbunden oder verbindbar sind,
**dadurch gekennzeichnet,**
**dass** der Steckerabschnitt (110) als einen selbsttragenden Bereich seiner selbst eine Manschette (140) aus einem elastischen Dichtungsmaterial aufweist, deren proximale Stirnwand eine senkrecht zur Axialrichtung der Manschette (140) stehende Dichtfläche bildet.

14. Elektrodenanordnung (100) nach einem der Ansprüche 11 bis 13,
**dadurch gekennzeichnet,**
**dass** die Manschette (140) die Form eines dickwandigen Hohlzylinders aufweist.

15. Elektrodenanordnung (100) nach einem der Ansprüche 11 bis 14,
**dadurch gekennzeichnet,**
**dass** zwei der steckerseitigen Kontaktelemente (123, 133) axial voneinander beabstandet sind und die Manschette (140) axial zwischen ihnen angeordnet ist.

16. Elektrodenanordnung (100) nach einem der Ansprüche 11 bis 15,
**dadurch gekennzeichnet,**
**dass** zwei der steckerseitigen Kontaktelemente (123', 123") azimutal voneinander beabstandet und proximal der Manschette (140) angeordnet sind,
wobei materialeinheitlich an die Manschette (140) zwei Stege (141) angeformt sind, die sich azimutal beidseitig zwischen besagten steckerseitigen Kontaktelementen (123', 123') axial bis zum proximalen Ende des Steckerabschnitts (110) erstrecken und dieses ineinander übergehend umlaufen.

17. Elektrodenanordnung (100) nach einem der Ansprüche 11 bis 15,
**dadurch gekennzeichnet,**
**dass** eines der steckerseitigen Kontaktelemente (123) als eine koaxiale und in einer Öffnung am proximalen Ende der Elektrodenanordnung (100) mündende Buchse ausgebildet ist.

18. Basisgerät (200) für ein elektrochirurgisches Instrument (10), umfassend eine die Form eines gerade erstreckten Kanals aufweisende Aufnahme (210) für einen Steckerabschnitt (110) einer Elektrodenanordnung (100),
wobei die Aufnahme (210) beabstandet voneinander eine Mehrzahl gegeneinander elektrisch isolierter, aufnahmeseitiger Kontaktelemente (221, 231) trägt, die über gegeneinander elektrisch isolierte Kontaktzuleitungen (222, 232) mit vom Äußeren des Basisgerätes (200) her zugänglichen Versorgungsanschlüssen verbunden oder verbindbar sind,
**dadurch gekennzeichnet,**
**dass** die Aufnahme (210) als proximale Begrenzung eines Dichtabschnitts (212), der zur dichtenden, radialen Anlage einer einen Bestandteil der Elektrodenanordnung (100) bildenden Manschette (140) aus einem elastischen Dichtungsmaterial geeignet ist, eine quer zu ihrer Axialrichtung erstreckte und nach distal weisende Anlagefläche (214) zur dichtenden, axialen Anlage der proximalen Stirnwand besagter Manschette (140) aufweist.

19. Basisgerät (200) nach Anspruch 18,
**dadurch gekennzeichnet,**
**dass** die Aufnahme (210) wenigstens in dem Dichtabschnitt (212) einen runden Querschnitt axial konstanten Durchmessers aufweist.

20. Basisgerät (200) nach einem der Ansprüche 18 bis 19,
**dadurch gekennzeichnet,**
**dass** zwei der aufnahmeseitigen Kontaktelemente (221, 231) axial voneinander beabstandet sind und der Dichtabschnitt (212) axial zwischen ihnen angeordnet ist.

21. Basisgerät (200) nach einem der Ansprüche 18 bis 20,
**dadurch gekennzeichnet,**
**dass** zwei der aufnahmeseitigen Kontaktelemente azimutal voneinander beabstandet und proximal des Dichtabschnitts angeordnet sind.

22. Basisgerät (200) nach einem der Ansprüche 18 bis 21,
**dadurch gekennzeichnet,**
**dass** eines der aufnahmeseitigen Kontaktelemente als ein koaxial von proximal her in den Innenraum der Aufnahme, insbesondere deren Dichtabschnitt, ragender Stift ausgebildet ist.

23. Basisgerät (200) nach einem der Ansprüche 18 bis 22,
**gekennzeichnet durch**
ein bewegbares, außerhalb des Dichtabschnitts (212) in den Innenraum der Aufnahme (210) ragendes Riegelelement.

24. Basisgerät (200) nach Anspruch 23,
**dadurch gekennzeichnet,**
**dass** das Riegelelement nach innen federvorgespannt ist.

25. Basisgerät (200) nach einem der Ansprüche 23 bis 24,
**dadurch gekennzeichnet,**
**dass** das Riegelelement eines der aufnahmeseitigen Kontaktelemente (221, 231) bildet.

26. Basisgerät (200) nach einem der Ansprüche 23 bis 25,
**dadurch gekennzeichnet,**
**dass** das Riegelelement radial und/oder tangential verschieblich und/oder verdrehbar ausgebildet ist.

## Claims

1. Electrosurgical instrument (10) comprising
- a base unit (200) having a receptacle (210) in the form of a straight channel for a plug section (110) of an electrode assembly (100), and
- an electrode assembly (100) with a rod assembly extending between a distal and a proximal end, the distal end of which rod assembly carries an electrosurgical effector or can be coupled to such an effector and the proximal end of which rod assembly is designed as a plug section (110) and is plugged into the receptacle (210) of the base unit (200) in a sealing manner,
wherein the receptacle (210) of the base unit (200) and the plug section (110) of the electrode assembly (100) each carry a plurality of spaced-apart contact elements (123, 123', 123", 133; 221, 231) which are electrically insulated from one another and form a corresponding plurality of contact pairs, each comprising a plug-side and a receptacle-side contact element (123, 123', 123", 133; 221, 231) which make electrical contact with one another,
and wherein the plug section (110) has a sleeve (140) made of an elastic sealing material, the outer wall of which sealingly abuts against the inner wall of a sealing section (212) of the receptacle (210),
**characterized in**
**that** the sleeve (140) is clamped axially between a plug-side fixing and a receptacle-side contact surface (214) , which extends transversely to the axial direction of the receptacle (210) and faces distally.

2. Electrosurgical instrument (10) according to claim 1,
**characterized in**
**that** the inner wall of the sleeve (140) rests sealingly against the outer wall of a supporting region of the plug section (110).

3. Electrosurgical instrument (10) according to claim 1,
**characterized in**
**that** the sleeve (140) is designed as a self-supporting region of the plug section (110).

4. Electrosurgical instrument (10) according to one of the preceding claims,
**characterized in**
**that** the sleeve (140) has the shape of a thick-walled hollow cylinder and the receptacle (210) has a round cross-section of axially constant diameter at least in the region of the sealing portion (212).

5. Electrosurgical instrument (10) according to one of the preceding claims,
**characterized in**
**that** the plug section (110) has an undercut with a distally facing stop against which a movable locking element rests, which projects into the interior of the receptacle.

6. Electrosurgical instrument (10) according to claim 5,
**characterized in**
**that** the undercut tapers distally in the form of a tapered bevel.

7. Electrosurgical instrument (10) according to any one of claims 5 to 6,
**characterized in**
**that** the undercut and the locking element are formed as the contact elements (231, 133) of one of the contact pairs.

8. Electrosurgical instrument (10) according to one of the preceding claims,
**characterized in**
**that** two of the contact pairs are axially spaced from each other and the sleeve (140) is arranged axially between them.

9. Electrosurgical instrument (10) according to one of the preceding claims,
**characterized in**
**that** two of the contact pairs are spaced azimuthally from one another and are arranged proximally of the sleeve (140),
wherein two webs (141) are moulded onto the sleeve (140) in the same material, the outer ridges of which rest against the inner wall of the receptacle (210) and extend azimuthally on both sides between the contact pairs axially as far as the proximal end of the plug section (110) and run around the latter merging into one another.

10. Electrosurgical instrument (10) according to any one of claims 1 to 8,
**characterized in**
**that** one of the contact pairs comprises a pin projecting coaxially from proximally into the interior of the receptacle (210), in particular its sealing portion (212), as the receptacle-side contact element and a corresponding, coaxial socket opening into an opening at the proximal end of the electrode arrangement as the plug-side contact element (123).

11. Electrode assembly (100) for an electrosurgical instrument (10), comprising a rod assembly extending between a distal and a proximal end, the distal end of which rod assembly carries an electrosurgical effector or can be coupled to such an end effector and the proximal end of which rod assembly is designed as a plug section (110) which carries a plurality of mutually electrically insulated, plug-side contact elements (123, 123', 123", 133) spaced apart from each other, which are connected or can be connected to electrodes of the effector via mutually electrically insulated electrode leads (124, 134), wherein the plug section (110) carries a sleeve (140) made of an elastic sealing material, the inner wall of which rests against the outer wall of a supporting region of the plug section (110),
**characterized in**
**that** the sleeve (140) rests distally against a plug-side abutment (131) and the proximal end wall of the sleeve (140) forms a sealing surface perpendicular to its axial direction.

12. Electrode assembly (100) according to claim 11,
**characterized in**
**that** the sleeve (140) is arranged axially displaceably on the plug section (110).

13. Electrode assembly (100) for an electrosurgical instrument (10), comprising a rod assembly extending between a distal and a proximal end, the distal end of which rod assembly carries an electrosurgical effector or can be coupled to such an end effector and the proximal end of which rod assembly is designed as a plug section (110) which carries a plurality of mutually electrically insulated, plug-side contact elements (123, 123', 123", 133) spaced apart from each other, which are connected or can be connected to electrodes of the effector via mutually electrically insulated electrode leads (124, 134), wherein the plug section (110) carries a sleeve (140) made of an elastic sealing material,
**characterized in**
**that** the sleeve (140) is designed as a self-supporting region of the plug section (110) and the proximal end wall of the sleeve (140) forms a sealing surface perpendicular to the axial direction of the sleeve (140).

14. Electrode assembly (100) according to any one of claims 11 to 13,
**characterized in**
**that** the sleeve (140) has the shape of a thick-walled hollow cylinder.

15. Electrode assembly (100) according to any one of claims 11 to 14,
**characterized in**
**that** two of the plug-side contact elements (123, 133) are axially spaced apart from one another and the sleeve (140) is arranged axially between them.

16. Electrode assembly (100) according to any one of claims 11 to 15,
**characterized in**
**that** two of the plug-side contact elements (123', 123") are spaced azimuthally from one another and are arranged proximally of the sleeve (140),
wherein two webs (141) are moulded onto the sleeve (140) in the same material, which webs extend azimuthally on both sides between said plug-side contact elements (123', 123') axially to the proximal end of the plug section (110) and run around the latter, merging into one another.

17. Electrode assembly (100) according to any one of claims 11 to 15,
**characterized in**
one of the plug-side contact elements (123) is designed as a coaxial socket opening into an opening at the proximal end of the electrode assembly (100).

18. Base unit (200) for an electrosurgical instrument (10), comprising a receptacle (210) in the form of a straight channel for a plug section (110) of an electrode assembly (100),
wherein the receptacle (210) carries a plurality of mutually electrically insulated, receptacle-side contact elements (221, 231) which are connected or connectable via mutually electrically insulated contact leads (222, 232) to supply terminals accessible from the exterior of the base unit (200),
**characterized in**
**that** the receptacle (210), as the proximal boundary of a sealing portion (212) which is suitable for the sealing, radial contact of a sleeve (140) made of an elastic sealing material and forming a component of the electrode assembly (100), has a contact surface (214) which extends transversely with respect to its axial direction and faces distally for the sealing, axial contact of the proximal end wall of said sleeve (140).

19. Base unit (200) according to claim 18,
**characterized in**
**that** the receptacle (210) has a round cross-section of axially constant diameter at least in the sealing portion (212).

20. Base unit (200) according to any one of claims 18 to 19,
**characterized in**
**that** two of the receptacle-side contact elements (221, 231) are axially spaced from each other and the sealing portion (212) is arranged axially between them.

21. Base unit (200) according to any one of claims 18 to 20,
**characterized in**
**that** two of the receptacle-side contact elements are azimuthally spaced from each other and arranged proximal to the sealing portion.

22. Base unit (200) according to any one of claims 18 to 21,
**characterized in**
**that** one of the receptacle-side contact elements is designed as a pin projecting coaxially from proximally into the interior of the receptacle, in particular its sealing portion.

23. Base unit (200) according to any one of claims 18 to 22,
**characterized by**
a movable latch element projecting outside the sealing portion (212) into the interior of the receptacle (210).

24. Base unit (200) according to claim 23,
**characterized in**
**that** the locking element is spring-biased inwards.

25. Base unit (200) according to any one of claims 23 to 24,
**characterized in**
**that** the latch element forms one of the receptacle-side contact elements (221, 231).

26. Base unit (200) according to any one of claims 23 to 25,
**characterized in**
**that** the locking element is designed to be radially and/or tangentially displaceable and/or rotatable.

## Revendications

1. Instrument électrochirurgical (10) comprenant
- un appareil de base (200) ayant un réceptacle (210) en forme de canal droit pour une section de fiche (110) d'un ensemble d'électrodes (100) et
- un ensemble d'électrodes (100) avec une tringlerie s'étendant entre une extrémité distale et une extrémité proximale, dont l'extrémité distale porte un effecteur électrochirurgical ou peut être couplée à un tel effecteur et dont l'extrémité proximale est réalisée sous la forme d'une section de fiche (110) et est enfichée de manière étanche dans le réceptacle (210) de l'appareil de base (200),
dans lequel le réceptacle (210) de l'appareil de base (200) et la section de fiche (110) de l'ensemble d'électrodes (100) portent chacun une pluralité d'éléments de contact (123, 123', 123", 133 ; 221, 231) espacés les uns des autres et isolés électriquement les uns des autres, qui forment une pluralité correspondante de paires de contacts comprenant chacune un élément de contact côté fiche et un élément de contact côté réceptacle (123, 123', 123", 133 ; 221, 231) qui sont en contact électrique les uns avec les autres,
et dans lequel la section de fiche (110) comprend une manchette (140) en matériau d'étanchéité élastique dont la paroi extérieure s'applique de manière étanche contre la paroi intérieure d'une section d'étanchéité (212) du réceptacle (210),
**caractérisé en ce**
**que** la manchette (140) est serrée axialement entre une fixation côté fiche et une surface d'appui (214) côté réceptacle, s'étendant transversalement à la direction axiale du réceptacle (210) et orientée vers le côté distal.

2. Instrument électrochirurgical (10) selon la revendication 1,
**caractérisé en ce**
**que** la paroi intérieure de la manchette (140) est en contact étanche avec la paroi extérieure d'une zone portante de la section de fiche (110).

3. Instrument électrochirurgical (10) selon la revendication 1,
**caractérisé en ce**
**que** la manchette (140) est conçue comme une zone autoportante de la section de fiche (110).

4. Instrument électrochirurgical (10) selon l'une des revendications précédentes,
**caractérisé en ce**
**que** la manchette (140) présente la forme d'un cylindre creux à paroi épaisse et le réceptacle (210) présente, au moins dans la zone de la section d'étanchéité (212), une section transversale ronde de diamètre axialement constant.

5. Instrument électrochirurgical (10) selon l'une des revendications précédentes,
**caractérisé en ce**
**que** la section de fiche (110) présente une contre-dépouille avec une butée orientée vers le côté distal, contre laquelle s'appuie un élément de verrouillage mobile faisant saillie dans l'espace intérieur du réceptacle.

6. Instrument électrochirurgical (10) selon la revendication 5,
**caractérisé en ce**
**que** la contre-dépouille se termine vers le côté distal sous la forme d'un biseau d'attaque.

7. Instrument électrochirurgical (10) selon l'une des revendications 5 à 6,
**caractérisé en ce**
**que** la contre-dépouille et l'élément de verrouillage sont conçus comme les éléments de contact (231, 133) de l'une des paires de contacts.

8. Instrument électrochirurgical (10) selon l'une des revendications précédentes,
**caractérisé en ce**
**que** deux des paires de contacts sont espacées axialement l'une de l'autre et la manchette (140) est disposée axialement entre elles.

9. Instrument électrochirurgical (10) selon l'une des revendications précédentes,
**caractérisé en ce**
**que** deux des paires de contacts sont disposées à une distance azimutale l'une de l'autre et à proximité de la manchette (140),
deux barrettes (141) étant formées sur la manchette (140) en un seul matériau, dont les bavures extérieures s'appliquent contre la paroi intérieure du réceptacle (210) et s'étendent en azimut de part et d'autre entre les paires de contacts, axialement jusqu'à l'extrémité proximale de la section de fiche (110) et entourent celle-ci en se confondant.

10. Instrument électrochirurgical (10) selon l'une quelconque des revendications 1 à 8,
**caractérisé en ce**
**que** l'une des paires de contacts est constituée d'une broche faisant saillie coaxialement depuis le côté proximal dans l'espace intérieur du réceptacle (210), en particulier de sa section d'étanchéité (212), en tant qu'élément de contact côté réceptacle, et d'une douille correspondante, coaxiale et débouchant dans une ouverture à l'extrémité proximale de l'ensemble d'électrodes, en tant qu'élément de contact côté fiche (123).

11. Ensemble d'électrodes (100) pour un instrument électrochirurgical (10), comprenant une tringlerie s'étendant entre une extrémité distale et une extrémité proximale, dont l'extrémité distale porte un effecteur électrochirurgical ou peut être couplée à un tel effecteur et dont l'extrémité proximale est réalisée sous la forme d'une section de fiche (110) qui porte, à distance les uns des autres, une pluralité d'éléments de contact côté fiche (123, 123', 123", 133) isolés électriquement les uns des autres, qui sont ou peuvent être reliés à des électrodes de l'effecteur par l'intermédiaire de lignes d'alimentation en électrodes (124, 134) isolées électriquement les unes des autres, la section de fiche (110) portant une manchette (140) en un matériau d'étanchéité élastique, dont la paroi intérieure s'applique contre la paroi extérieure d'une zone portante de la section de fiche (110),
**caractérisé en ce**
**que** la manchette (140) s'applique distalement contre un appui côté fiche (131) et la paroi frontale proximale de la manchette (140) forme une surface d'étanchéité perpendiculaire à sa direction axiale.

12. Ensemble d'électrodes (100) selon la revendication 11,
**caractérisé en ce**
**que** la manchette (140) est disposée de manière à pouvoir se déplacer axialement sur la section de connecteur (110).

13. Ensemble d'électrodes (100) pour un instrument électrochirurgical (10), comprenant une tringlerie s'étendant entre une extrémité distale et une extrémité proximale, dont l'extrémité distale porte un effecteur électrochirurgical ou peut être couplée à un tel effecteur et dont l'extrémité proximale est réalisée sous la forme d'une section de fiche (110) qui porte, à distance les uns des autres, une pluralité d'éléments de contact côté fiche (123, 123', 123", 133) isolés électriquement les uns des autres, qui sont ou peuvent être reliés à des électrodes de l'effecteur par l'intermédiaire de lignes d'alimentation en électrodes (124, 134) isolées électriquement les unes des autres, la section de fiche (110) portant une manchette (140) en un matériau d'étanchéité élastique,
**caractérisé en ce**
**que** la manchette (140) est réalisée sous la forme d'une zone autoportante de la section de connecteur (110) et la paroi frontale proximale de la manchette (140) forme une surface d'étanchéité perpendiculaire à la direction axiale de la manchette (140).

14. Ensemble d'électrodes (100) selon l'une quelconque des revendications 11 à 13,
**caractérisé en ce**
**que** la manchette (140) a la forme d'un cylindre creux à paroi épaisse.

15. Ensemble d'électrodes (100) selon l'une quelconque des revendications 11 à 14,
**caractérisé en ce**
**que** deux des éléments de contact côté fiche (123, 133) sont espacés axialement l'un de l'autre et la manchette (140) est disposée axialement entre eux.

16. Ensemble d'électrodes (100) selon l'une quelconque des revendications 11 à 15,
**caractérisé en ce**
**que** deux des éléments de contact côté fiche (123', 123") sont disposées à une distance azimutale l'une de l'autre et à proximité de la manchette (140),
deux barrettes (141) étant formées sur la manchette (140) en un seul matériau et s'étendent en azimut de part et d'autre entre lesdits éléments de contact côté fiche (123', 123'), axialement jusqu'à l'extrémité proximale de la section de fiche (110) et entourent celle-ci en se confondant.

17. Ensemble d'électrodes (100) selon l'une quelconque des revendications 11 à 15,
**caractérisé en ce**
**que** l'un des éléments de contact côté fiche (123) est conçu comme une douille coaxiale et débouchant dans une ouverture à l'extrémité proximale de l'ensemble d'électrodes (100).

18. Appareil de base (200) pour un instrument électrochirurgical (10), comprenant un réceptacle (210) ayant la forme d'un canal droit pour une section de fiche (110) d'un ensemble d'électrodes (100),
le réceptacle (210) portant, à distance les uns des autres, une pluralité d'éléments de contact côté réceptacle (221, 231) isolés électriquement les uns des autres, qui sont reliés ou peuvent être reliés, par l'intermédiaire de lignes d'amenée de contact (222, 232) isolées électriquement les unes des autres, à des raccords d'alimentation accessibles depuis l'extérieur de l'appareil de base (200),
**caractérisé en ce**
**que** le réceptacle (210) présente, en tant que délimitation proximale d'une section d'étanchéité (212) qui est appropriée pour l'appui radial étanche d'une manchette (140) en un matériau d'étanchéité élastique formant un composant de l'ensemble d'électrodes (100), une surface d'appui (214) s'étendant transversalement à sa direction axiale et dirigée vers le côté distal pour l'appui axial étanche de la paroi frontale proximale de ladite manchette (140).

19. Appareil de base (200) selon la revendication 18,
**caractérisé en ce**
**que** le réceptacle (210) présente, au moins dans la section d'étanchéité (212), une section transversale circulaire de diamètre axialement constant.

20. Appareil de base (200) selon l'une quelconque des revendications 18 à 19,
**caractérisé en ce**
**que** deux des éléments de contact côté réceptacle (221, 231) sont espacés axialement l'un de l'autre et la section d'étanchéité (212) est disposée axialement entre eux.

21. Appareil de base (200) selon l'une quelconque des revendications 18 à 20,
**caractérisé en ce**
**que** deux des éléments de contact côté réceptacle sont espacés en azimut l'un de l'autre et sont disposés de manière proximale par rapport à la section d'étanchéité.

22. Appareil de base (200) selon l'une quelconque des revendications 18 à 21,
**caractérisé en ce**
**que** l'un des éléments de contact côté réceptacle est réalisé sous la forme d'une broche faisant saillie coaxialement depuis le côté proximal dans l'espace intérieur du réceptacle, en particulier dans sa section d'étanchéité.

23. Appareil de base (200) selon l'une des revendications 18 à 22,
**caractérisé par**
un élément de verrouillage mobile, dépassant à l'extérieur de la section d'étanchéité (212) dans l'espace intérieur du réceptacle (210).

24. Appareil de base (200) selon la revendication 23,
**caractérisé en ce**
**que** l'élément de verrouillage est précontraint par ressort vers l'intérieur.

25. Appareil de base (200) selon l'une quelconque des revendications 23 à 24,
**caractérisé en ce**
**que** l'élément de verrouillage forme l'un des éléments de contact côté réceptacle (221, 231).

26. Appareil de base (200) selon l'une quelconque des revendications 23 à 25,
**caractérisé en ce**
**que** l'élément de verrouillage est conçu de manière à pouvoir se déplacer radialement et/ou tangentiellement et/ou à pouvoir tourner.
